# EUROPÄISCHE PATENTSCHRIFT

(11) **EP 0 628 031 B1**
(45) Veröffentlichungstag und Bekanntmachung des Hinweises auf die Patenterteilung: **16.06.1999**
(21) Anmeldenummer: 93903974.9
(22) Anmeldetag: 16.02.1993
(51) Int. Cl.: C07D 239/47, A01N 47/36

(54) **HERBIZIDE N- (PYRIMIDIN-2-YL)AMINOCARBONYL]BENZOLSULFONAMIDE**
HERBICIDE N- (PYRIMIDIN-2-YL)AMINOCARBONYL] BENZENE SULPHONAMIDES
N- (PYRIMIDIN-2-YL)AMINOCARBONYL] SULFAMIDES BENZENIQUES HERBICIDES

(30) Priorität: 28.02.1992 DE 4206145
(43) Veröffentlichungstag der Anmeldung: 14.12.1994
(73) Patentinhaber: BASF Aktiengesellschaft, 67063 Ludwigshafen (DE)
(72) Erfinder: MAYER, Horst, D-6700 Ludwigshafen (DE); HAMPRECHT, Gerhard, D-6940 Weinheim (DE); WESTPHALEN, Karl-Otto, D-6720 Speyer (DE); GERBER, Matthias, D-6703 Limburgerhof (DE); KARDORFF, Uwe, D-6800 Mannheim 1 (DE); WALTER, Helmut, D-6719 Obrigheim (DE)
(86) Internationale Anmeldenummer: EP9300362
(87) Internationale Veröffentlichungsnummer: WO9316998

(56) Entgegenhaltungen:
- EP-A- 0 044 209
- EP-A- 0 101 308
- EP-A- 0 285 978
- EP-A- 0 378 092
- US-A- 4 169 719

## Beschreibung

Die vorliegende Erfindung betrifft N-[(Pyrimidin-2-yl)-aminocarbonyl]benzolsulfonamide der allgemeinen Formel I 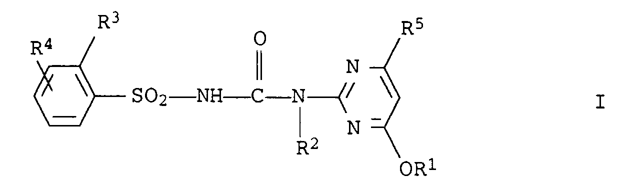 in der die Substituenten folgende Bedeutung haben:
- R¹: Methyl;
- R²: Wasserstoff;
- R³: Trifluormethyl oder Trifluormethoxy;
- R⁴: Wasserstoff;
- R⁵: Fluor oder Trifluormethyl
sowie deren umweltverträgliche Salze.

Des weiteren betrifft die Erfindung ein Verfahren zur Herstellung der Verbindungen I sowie ihre Verwendung als herbizide Mittel.

Dem Stand der Technik sind eine Reihe von Patentschriften zu entnehmen, die Sulfonylharnstoffe mit herbizider Wirkung zum Gegenstand haben.

In der EP-A 44 808 sind tabellarisch ohne nähere Charakterisierung die Verbindungen A - D aufgeführt.

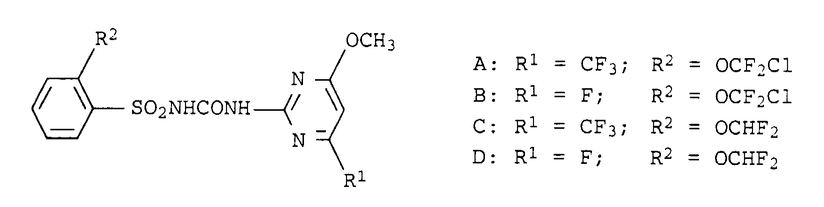

Ebenfalls ohne physikalische Kenndaten ist der Sulfonylharnstoff E in der EP-A 44 807 genannt.

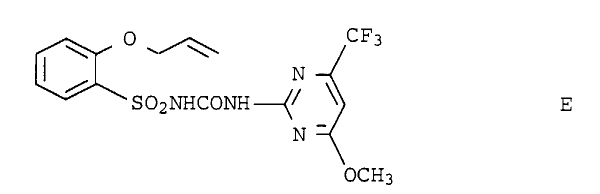

Die EP-A 338 424 beschreibt Benzoesäuremethylester wie z.B. F.

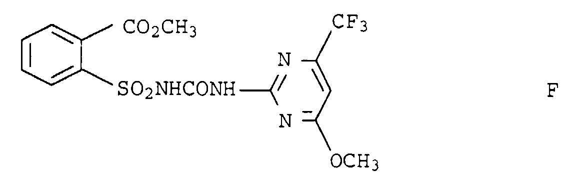

Gegenstand der DE-A 39 00 472 sind Benzoesäureester und Benzoesäureamide sowie o-Halogensubstituierte Sulfonylharnstoffe wie z.B. der Struktur G-I

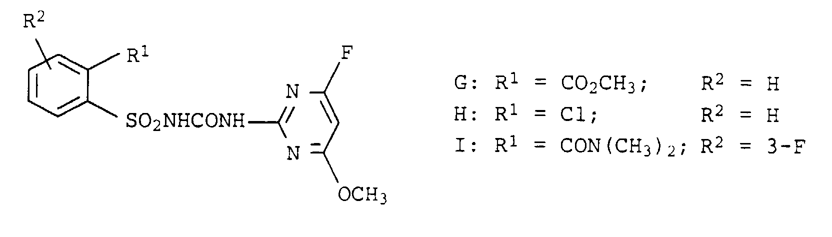

Die EP-A 101 308 beschreibt höhere Sulfone z.B: J-M

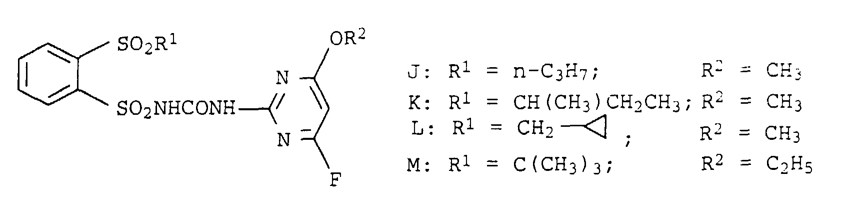

In der US 4 169 719, Kolonne 13, Zeile 59 ist der Sulfonylharnstoff N genannt.

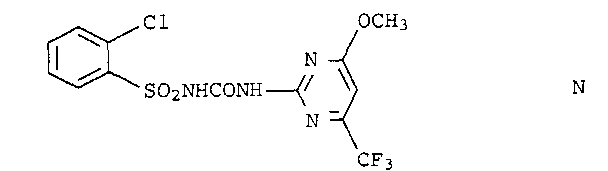

Das US-Patent 4 310 346 offenbart Sulfonamide der Struktur P.

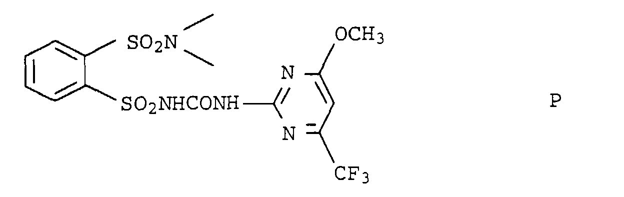

Dimethylcarbamoyl substituierte Sulfonylharnstoffe der Struktur R 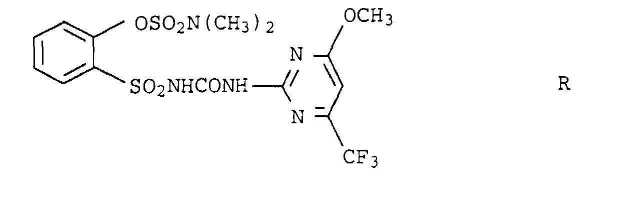 werden im US-Patent 4,515,624 offenbart.

Sulfonylharnstoffderivate mit Fluoralkoxysubstitution, Sulfamoylsubstitution, Acylsubstitution bzw. Alkylsubstitution in ortho-Stellung des Phenylrestes werden in den Veröffentlichungen EP-A 173 312, US-B 4 515 624, US-B 4 425 153 bzw. EP-A 44 209 von allgemeinen Strukturformeln umfaßt, ohne daß nähere Ausführungen zu konkreten Strukturen entnommen werden können.

Die älteren deutschen Anmeldungen P 40 38 430.6 vom 01.12.1990 und P 41 05 518.7 vom 22.02.1991 beschreiben herbizid wirksame Sulfonamide mit gegenüber den erfindungsgemäßen Verbindungen unterschiedlicher Substituenten in ortho-Position des Phenylrestes und/oder in 3/5-Position des Triazinrestes.

Der Erfindung lag nun die Aufgabe zugrunde, Sulfonylharnstoffe zu synthetisieren, die gegenüber den bekannten Vertretern dieser Herbizid-Klasse verbesserte Eigenschaften aufweisen.

Entsprechend dieser Aufgabe wurden die eingangs definierten N-[(Pyrimidin-2-yl)aminocarbonyl]benzolsulfonamide der Formel I gefunden.

Im Hinblick auf ihre bestimmungsgemäße Verwendung kommen als Substituenten beispielsweise folgende Reste in Betracht:
- R¹: Methyl oder Ethyl;
- R²: Wasserstoff oder Methyl;
- R³: Trifluormethyl oder Trifluormethoxy;
- R⁴: Wasserstoff;
- R⁵: Fluor oder Trifluormethyl.

Besonders bevorzugte Verbindungen I ergeben sich durch folgende Restekombinationen:
a) R¹ = Methyl, R², R⁴ = H, R³ = Trifluormethyl, R⁵ = Trifluormethyl oder Fluor;
b) R¹ = Methyl, R², R⁴ = H, R³ = Trifluormethoxy, R⁵ = Trifluormethyl oder Fluor;

Die erfindungsgemäßen Sulfonylharnstoffe der Formel I sind auf verschiedenen Wegen zugänglich, die in der Literatur beschrieben sind. Beispielhaft seien besonders vorteilhafte Wege (A-D) im folgenden näher erläutert. 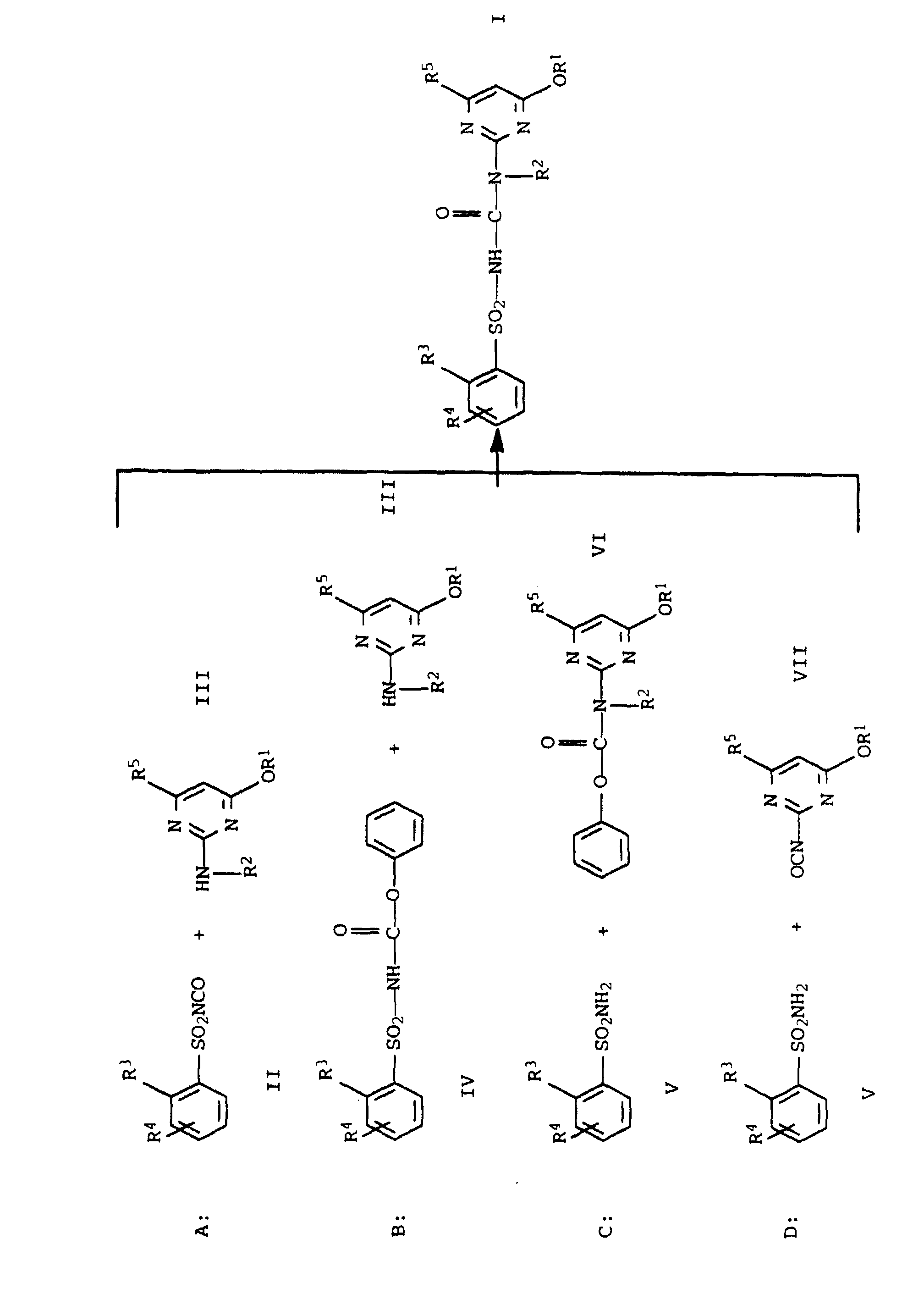
A: Man setzt ein Sulfonylisocyanat II in an sich bekannter Weise (EP-A-162 723) mit ungefähr der stöchiometrischen Menge eines 2-Amino-pyrimidinderivats III bei einer Temperatur von 0 bis 120°C, vorzugsweise 10 bis 100°C um. Die Reaktion kann unter Normaldruck oder unter Druck (bis 50 bar), vorzugsweise bei 1 bis 5 bar, kontinuierlich oder diskontinuierlich durchgeführt werden.
   Zweckmäßigerweise verwendet man für die Umsetzungen unter den jeweiligen Reaktionsbedingungen inerte Lösungs- und Verdünnungsmittel. Als Lösungsmittel kommen beispielsweise Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan,
   o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether,
   n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon und entsprechende Gemische in Betracht. Zweckmäßig verwendet man das Lösungsmittel in einer Menge von 100 bis 2000 Gew.%, vorzugsweise von 200 bis 700 Gew.%, bezogen auf den Ausgangsstoff II.
   Die zur Umsetzung benötigte Verbindung II wird im allgemeinen in etwa äquimolaren Mengen (mit einem Über- oder Unterschuß von z.B. 0 bis 20 %, bezogen auf den jeweiligen Ausgangsstoff III) eingesetzt. Man kann den Ausgangsstoff III in einem der vorgenannten Verdünnungsmittel vorlegen und dann den Ausgangsstoff II zugeben.
   Zweckmäßigerweise wird das Verfahren zur Herstellung der neuen Verbindungen jedoch so durchgeführt, daß man den Ausgangsstoff II, gegebenenfalls in einem der vorgenannten Verdünnungsmittel, vorlegt und dann den Ausgangsstoff III zugibt.
   Zur Beendigung der Umsetzung rührt man nach der Zugabe der Komponenten noch 20 Minuten bis 24 Stunden bei 0 bis 120°C, vorzugsweise 10 bis 100°C, nach.
   Als Reaktionsbeschleuniger kann man vorteilhafterweise ein tertiäres Amin, z.B. Pyridin, α,β,γ-Picolin, 2,4-, 2,6-Lutidin, 2,4,6-Collidin, p-Dimethylaminopyridin, Trimethylamin, Triethylamin, Tri(n-propyl)amin, 1,4-Diaza[2,2,2]bicyclooctan [DABCO] oder 1,8-Diazabicylco[5,4,0]-undec-7-en in einer Menge von 0.01 bis 1 Mol pro Mol Ausgangsstoff II verwenden.
   Aus dem Reaktionsgemisch wird der Endstoff I in üblicher Weise, z.B. nach Abdestillieren von Lösungsmittel oder direkt durch Absaugen isoliert. Der verbleibende Rückstand kann noch mit Wasser bzw. verdünnter Säure zur Entfernung basischer Verunreinigungen gewaschen werden. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Rühren in einem organischen Lösungsmittel, das die Verunreinigungen aufnimmt oder Chromatographie gereinigt werden.
   Bevorzugt führt man diese Umsetzung in Acetonitril, Methyltert.-butylether, Toluol oder Methylenchlorid in Anwesenheit von 0 bis 100 Moläquivalenten, vorzugsweise 0 bis 50 Moläquivalenten eines tertiären Amins wie 1,4-Diazabicyclo[2,2,2]octan oder Triethylamin durch.
B: Man setzt ein entsprechendes Sulfonylcarbamat der Formel IV in an sich bekannter Weise (EP-A-120 814, EP-A-101 407) in einem inerten organischen Lösungsmittel bei einer Temperatur zwischen 0 und 120°C, vorzugsweise 10 bis 100°C mit einem 2-Amino-pyrimidinderivat III um. Es können hierbei Basen wie tertiäre Amine zugesetzt werden, wodurch die Reaktion beschleunigt und die Produktqualität verbessert wird.
   Geeignete Basen hierfür sind z.B. tertiäre Amine wie unter A angegeben, insbesondere Triethylamin oder 1,4-Diazabicyclo[2,2,2]octan, in einer Menge von 0,01 bis 1 mol pro Mol Ausgangsstoff IV.
   Zweckmäßig verwendet man als Lösungsmittel die unter A angegebenen.
   Man verwendet das Lösungsmittel in einer Menge von 100 bis 2.000 Gew.%, vorzugsweise von 200 bis 700 Gew.%, bezogen auf den Ausgangsstoff IV.
   Die zur Umsetzung benötigte Verbindung IV wird im allgemeinen in etwa äquimolaren Mengen (mit einem Über- oder Unterschuß von z.B. 0 bis 20 %, bezogen auf jeweiligen Ausgangsstoff III eingesetzt. Man kann den Ausgangsstoff IV in einem der vorgenannten Verdünnungsmittel vorlegen und dann den Ausgangsstoff III zugeben.
   Man kann jedoch auch den Ausgangsstoff III in einem der genannten Löse- oder Verdünnungsmittel vorlegen und das Sulfonylcarbamat IV zugeben.
   In beiden Fallen kann als Katalysator vor oder während der Reaktion eine Base zugesetzt werden.
   Aus dem Reaktionsgemisch kann das Endprodukt I in üblicher Weise, wie unter A angegeben, gewonnen werden.
C: Man setzt ein Sulfonamid der Formel V in an sich bekannter Weise (EP-A-141 777 und EP-A-101 670) in einem inerten organischen Lösungsmittel mit ungefähr der stöchiometrischen Menge eines Phenylcarbamats VI bei einer Temperatur von 0 bis 120°C, vorzugsweise 20 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es können hierbei Basen wie tertiäre Amine zugesetzt werden, die die Reaktion beschleunigen und die Produktqualität verbessern. Geeignete Basen sind hierfür die unter A angegebenen, insbesondere Triethylamin, 2,4,6-Collidin, 1,4-Diazabicyclo[2,2,2]octan [DABCO] oder 1,8-Diazabicyclo-[5,4,0]undec-7-en (DBU), in einer Menge von 0,01 bis 1 mol pro Mol Ausgangsstoff V.
   Zweckmäßigerweise verwendet man als Löse- oder Verdünnungsmittel die unter A angegebenen.
   Man verwendet das Lösungsmittel in einer Menge von 100 bis 2000 Gew.%, vorzugsweise von 200 bis 700 Gew.%, bezogen auf das Edukt V.
   Die zur Umsetzung benötigte Verbindung V wird im allgemeinen in etwa äquimolaren Mengen (mit einem Über- oder Unterschuß von z.B. 0 bis 20 %, bezogen auf die jeweiligen Ausgangsstoffe VI) eingesetzt. Man kann den Ausgangsstoff VI in einem der vorgenannten Verdünnungsmittel vorlegen und dann den Ausgangsstoff V zugeben.
   Man kann jedoch auch den Ausgangsstoff V in einem der genannten Lösungsmittel vorlegen und dann das Carbamat VI zugeben. In beiden Fallen kann als Katalysator vor oder während der Reaktion eine der genannten Basen zugesetzt werden.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach.
   Man isoliert die Sulfonylharnstoffe der Formel I aus dem Reaktionsgemisch mit den üblichen Methoden, wie unter A beschrieben.
D: Man setzt ein Sulfonamid der Formel V in an sich bekannter Weise (EP-A-234 352) in einem inerten organischen Lösungsmittel mit ungefähr der stöchiometrischen Menge eines Isocyanates VII bei einer Temperatur von 0 bis 150°C, vorzugsweise 10 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es kannen hierbei vor oder während der Reaktion Basen wie tertiäre Amine zugesetzt werden, die die Reaktion beschleunigen und die Produktqualität verbessern. Geeignete Basen sind hierfür die unter A angegebenen, insbesondere Triethylamin oder 2,4,6-Collidin, in einer Menge von 0,01 bis 1 mol pro Mol Ausgangsstoff V.
   Zweckmäßigerweise verwendet man als Lösungsmittel die unter A angegebenen. Man setzt das Lösungsmittel in einer Menge von 100 bis 2000 Gew.%, vorzugsweise von 200 bis 700 Gew.%, bezogen auf das Edukt V ein.
   Die zur Umsetzung benötigte Verbindung V wird im allgemeinen in etwa äquimolaren Mengen (mit einem Über- oder Unterschuß von z.B. 0 bis 20 %, bezogen auf die Edukte VII) eingesetzt. Man kann den Ausgangsstoff VII in einem der genannten Verdünnungsmittel vorlegen und dann den Ausgangsstoff V zugeben. Man kann aber auch das Sulfonamid vorlegen und dann das Isocyanat VII zugeben.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach. Das Endprodukt I kann aus dem Reaktionsgemisch in der üblichen Weise, wie unter A: beschrieben, gewonnen werden.

Die als Ausgangsstoffe benötigten Sulfonylisocyanate der Formel II lassen sich in an sich bekannter Weise aus den entsprechenden Sulfonamiden durch Phosgenierung (Houben-Weyl 11/2 (1985) 1106, US 4 379 769) oder durch Umsetzung der Sulfonamide mit Chlorsulfonylisocyanat (DE-OS 3 132 944) gewinnen.

Die Sulfonamide der Formel V lassen sich durch Reaktion der entsprechenden Sulfonsäurechloride mit Ammoniak gewinnen (M. Quaedvlieg in Houben-Weyl, "Methoden der organischen Chemie", Georg Thieme Verlag, Stuttgart, Bd. 9 (1955) 398-400, F. Muth, ibid., 605ff.). Man kann jedoch auch in einer nukleophilen Substitution ein o-Halogenbenzolsulfonamid z.B. mit einem Alkohol oder Thiol umsetzen und beispielsweise den entsprechenden Thioether zum Sulfoxid oder Sulfon oxydieren (s. Verfahrensbeispiele).

Die entsprechenden Sulfonsäurechloride zur Herstellung der Sulfonamide der Formel V erhält man allgemein durch Meerwein-Reaktion (Diazotierung geeigneter Amine und Kupfersalz-katalysierte Sulfochlorierung mit Schwefeldioxid: F. Muth in Houben-Weyl, "Methoden der organischen Chemie", Georg Thieme Verlag, Stuttgart, Bd. 9 (1955) 579, S. Pawlenko in Houben-Weyl, "Methoden der organischen Chemie", Georg Thieme Verlag, Stuttgart, Bd. E 11/2 (1985) 1069), aus den entsprechenden Sulfonsäuren (F. Muth in Houben-Weyl, "Methoden der organischen Chemie", Georg Thieme Verlag, Stuttgart, Bd. 9 (1955) 564), durch Chlorsulfonierung geeigneter aromatischer Vorstufen (F. Muth, ibid., S. 572) oder durch oxydative Chlorierung niederwertiger Schwefelvorstufen (Mercaptane, Diaryldisulfide, s-Benzylmercaptane (F. Muth, ibid., S. 580, S. Pawlenko, loc. cit., S. 1073).

Die Sulfonylcarbamate der Formel IV wurden nach oder in Analogie zu an sich bekannten Reaktionen (z.B. EP-A 120 814) hergestellt. Man kann aber auch die Sulfonylisocyanate der Formel II in einem inerten Lösungsmittel wie Ether oder Dichlormethan mit Phenol in die Carbamate der Formel IV überführen.

Carbamate der Formel VI sind nach oder in Analogie zu bekannten Umsetzungen (z.B. EP-A 101 670) zugänglich, sie lassen sich aber auch aus den entsprechenden Isocyanaten VII durch Umsetzung mit Phenol herstellen.

Die Isocyanate der Formel VII erhält man aus den Aminen der Formel III durch Behandlung mit Oxalylchlorid oder Phosgen (in Analogie nach Angew. Chem. 83 (1971) 407, EP-A 388 873).

Die Synthese von 2-Amino-4-fluor-6-methoxypyrimidin und 2-Amino-4-ethoxy-6-fluorpyrimidin ist in der DE-A-39 00 471 offenbart.

2-Amino-4-chlor-6-trifluormethylpyrimidin ist literaturbekannt (J. Heterocycl. Chem. 20 (1983) 219). Aus diesem Vorprodukt lassen sich die 4-Alkoxy-2-amino-6-trifluormethylpyrimidine III (R¹ = Methyl, Ethyl; R² = H) durch Umsetzung mit den entsprechenden Alkoholaten gewinnen (s. Verfahrensbeispiel).

4-Methoxy-2-methylmercapto-6-trifluorpyrimidin (J. Heterocycl. Chem. 20 (1983) 219) läßt sich durch H₂O₂ in das 2-Methylsulfon überführen, das durch Umsetzung mit Aminen und Hydroxylaminen die Ausgangsstoffe der allgemeinen Formel III liefert (s. Verfahrensbeispiele).

Die Salze der Verbindungen I sind in an sich bekannter Weise zugänglich (EP-A-304 282, US-A 4,599,412). Man erhält sie durch Deprotonierung der entsprechenden Sulfonylharnstoffe I in Wasser oder einem inerten organischen Lösungsmittel bei Temperaturen von -80°C bis 120°C, vorzugsweise 0°C bis 60°C in Gegenwart einer Base.

Geeignete Basen sind beispielsweise Alkali- oder Erdalkalimetallhydroxide, -hydride, -oxide oder -alkoholate wie Natrium-, Kalium- und Lithiumhydroxid, Natriummethanolat, -ethanolat und - tert.-butanolat, Natrium- und Calziumhydrid und Calziumoxid.

Als Lösungsmittel kommen beispielsweise neben Wasser auch Alkohole wie Methanol, Ethanol und tert.-Butanol, Ether wie Tetrahydrofuran und Dioxan, Acetonitril, Dimethylformamid, Ketone wie Aceton und Methylethylketon und auch halogenierte Kohlenwasserstoffe in Betracht.

Die Deprotonierung kann bei Normaldruck oder bei Drucken bis 50 bar, vorzugsweise bei Normaldruck bis 5 bar Überdruck durchgeführt werden.

Die Verbindungen I bzw. die sie enthaltenden herbiziden Mittel sowie deren umweltverträgliche Salze von Alkalimetallen und Erdalkalimetallen können in Kulturen wie Weizen, Reis und Mais Schadpflanzen sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff.

Beispiele für Formulierungen sind:
- I.: Man vermischt 90 Gewichtsteile einer Verbindung der Formel I mit 10 Gewichtsteilen N-Methyl-α-pyrrolidon und erhält eine Lösung, die zur Anwendung in Form kleinster Tropfen geeignet ist.
- II.: 20 Gewichtsteile einer Verbindung der Formel I werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
- III.: 20 Gewichtsteile einer Verbindung der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
- IV.: 20 Gewichtsteile eines Wirkstoffs der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
- V.: 20 Gewichtsteile eines Wirkstoffs der Formel I werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
- VI.: 3 Gewichtsteile eines Wirkstoffs der Formel I werden mit 97 Gewichtsteilen feinteiligem Kaolin vermischt. Man erhält auf diese Weise ein Stäubemittel, das 3 Gew.% des Wirkstoffs enthält.
- VII.: 30 Gewichtsteile eines Wirkstoffs der Formel I werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
- VIII.: 20 Gewichtsteile eines Wirkstoffs der Formel I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3 kg/ha, vorzugsweise 0,01 bis 1 kg/ha aktive Substanz.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Citrus limon | Zitrone |
| Citrus sinensis | Apfelsine, Orange |
| Coffea arabica (Coffea canephora, | Kaffee |
| Coffea liberica) | |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Solanum tuberosum | Kartoffel |
| Sorghum bicolor (S. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Pyrimidinyl-substituierten Sulfonylharnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Nachstehend sind typische Beispiele zur Herstellung der Vorprodukte II-VII wiedergegeben.

### 1. 2-Amino-4-methoxy-6-trifluormethylpyrimidin

Zu einer Lösung von 25,7 g 2-Amino-4-chlor-6-trifluormethylpyrimidin (0,13 mol) in 200 ml Methanol tropfte man 28,1 g einer 30 gew.-%igen Lösung von Natriummethanolat (0,16 mol) in Methanol (Innentemperatur unter 40°C). Man rührte eine Stunde bei 20 - 25°C, entfernte die flüchtigen Anteile im Wasserstrahlvakuum bei 65°C, nahm den Rückstand in 11 Dichlormethan auf, wusch die Lösung mit Wasser, trocknete die Dichlormethanphase über Na₂SO₄ und entfernte das Lösungsmittel im Wasserstrahlvakuum. Man erhielt 23,4 g des Produktes (93 % d. Th.) als farblose Kristalle mit Fp. 108 - 109°C.

### 2. 4-Methoxy-2-methylsulfonyl-1-trifluormethylpyrimidin

Zu einer Lösung von 5,0 g 2,4-Methoxy-2-methylsulfonyl-6-trifluormethylpyrimidin (22 mmol) in 50 ml Eisessig tropfte man 7,1 g einer 30 gew.-%igen Lösung von H₂O₂ (63 mmol) in Wasser. Man erhitzte langsam auf eine Innentemperatur von 90°C, rührte 1 Stunde bei dieser Temperatur und trug den Reaktionsansatz nach Abkühlen auf 25°C in 2 1 Eisessig ein. Man extrahierte das Produkt mit 200 ml Dichlormethan, wusch die organische Phase mit einer verdünnten Natriumthiosulfatlösung, dann mit Wasser, trocknete die Lösung des Produkts über Na₂SO₄ und entfernte das Lösungsmittel im Wasserstrahlvakuum. Man erhielt 5,1 g des Produktes (90 % d. Th.) als farblose Kristalle mit Fp. 88-90°C.

### 3. 2-(N-Methoxyamino)-4-methoxy-6-trifluormethylpyrimidin

Eine Lösung von 6,5 g O-Methylhydroxylammoniumchlorid (78 mmol) in 200 ml Tetrahydrofuran wurde bei -10°C tropfenweise mit 66,6 g einer 1,6 molaren Lösung von n-Butyllithium (0,156 mol) in n-Hexan versetzt. Man rührte 10 Minuten bei -10°C und versetzte die trübe Lösung mit 20 g 4-Methoxy-2-methylsulfonyl-6-trifluormethylpyrimidin (78 mmol). Man rührte 1 Stunde bei 25°C, trug den Reaktionsansatz in 200 ml Wasser ein, trennte die organische Phase ab, wusch diese mit Wasser und trocknete diese. Nach Entfernen des Lösungsmittels verblieb ein öliges, leicht verunreinigtes Produkt, das in Pentan bei -78°C kristallisiert werden konnte. Man erhielt 7,5 g des Produktes (43 % d. Th.) als farblose Kristalle mit Fp. 44-46°C.

### 4. 2-Isocyanato-4-methoxy-6-trifluormethylpyrimidin

Zu einer Suspension von 2-Amino-4-methoxy-6-trifluormethylpyrimidin (78 mmol) in 100 ml Toluol tropfte man 38,3 g Oxalylchlorid (0,3 mol). Man rührte 4 Stunden unter Rückfluß, wonach eine homogene Lösung vorlag, die fraktioniert destilliert wurde. Man erhielt die Titelverbindung (9,9 g; 58 % d. Th.) als Öl mit Kp. 44-50°C (0,5 mbar).

### 5. 4-Methoxy-2-[(phenoxycarbonyl)amino]-6-trifluormethylpyrimidin

Zu einer Lösung von 9,9 g 2-Isocyanato-4-methoxy-6-trifluormethylpyrimidin (45 mmol) in 50 ml Methylenchlorid gab man bei 25°C 4,2 g Phenol (45 mmol). Man rührte 16 Stunden bei 25°C, destillierte das Methylenchlorid unter vermindertem Druck ab und rührte den Rückstand kräftig mit 100 ml eines Diisopropylether-/Hexan-Gemisches (v/v 1:20), worauf Kristallisation eintrat. Das Produkt wurde abgesaugt und im Wasserstrahlvakuum bei 50°C getrocknet. Man erhielt die Titelverbindung (12,2 g; 87 % d. Th.) als farblose Kristalle mit Fp. 85 - 88°C.

### 6. 2-(Methylsulfinyl)benzolsulfonamid

Zu einer Suspension von 26,5 g 2-(Methylthio)benzolsulfonamid (0,13 mol) und 2,1 g Na₂WO₄·2 H₂O in 88 ml Eisessig tropfte man bei einer Temperatur zwischen 25 und 30°C 14,8 g Wasserstoffperoxid (0,13 mol) 30 % in H₂O. Man rührte 45 min bei 25°C, goß das Reaktionsgemisch auf 400 ml Wasser und saugte den Niederschlag ab. Dieser wurde mit Wasser gewaschen und im Wasserstrahlvakuum bei 40°C getrocknet. Man erhielt so 24,3 g (85 % d. Th.) der Titelverbindung.
¹H-NMR-Spektrum (250 MHz, CD₃SOCD₃, int. TMS): 8,16 d (1H), 7,82-8,0 m (2H), 7,77 br (2H), 7.63-7.85 m (2H), 2,76 s (3H).

### 7. N-(n-Butylamino)carbonyl-2-methylsulfinylbenzolsulfonamid

Zu einer Suspension von 20,1 g 2-(Methylsulfinyl)benzolsulfonamid (0,09 mol) in 250 ml Acetonitril tropfte man bei 25°C 10,2 g n-Butylisocyanat (0,10 mol). Nach Zugabe von 13,9 g Kaliumcarbonat (0,10 mol) rührte man 4 h unter Rückfluß. Nach Abkühlen auf 0°C goß man auf 400 ml Eis/Wasser, stellte durch Zugabe von konz. Salzsäure einen pH-Wert von 1 ein und extrahierte mit Methylenchlorid. Die organischen Extrakte wurden mit Wasser neutral gewaschen und getrocknet. Nach Entfernen des Lösungsmittels erhielt man 25 g der Titelverbindung (85 % d. Theorie) als hellbraunes Öl.
¹H-NMR-Spektrum (250 MHz, CDCl₃, int. TMS): 8,28 d (1H), 8,20 d (1H), 7,89 t (2H), 7.73 t (1H), 6,03 t (1H), 3,13 m (2H), 2,95 s (3H), 1,38 m (2H), 1,24 m (2H), 0,85 t (3H).

### 8. 2-(Methylsulfinyl)benzolsulfonylisocyanat

In eine Lösung von 25 g N-[(n-Butylamino)carbonyl-2-methylsulfinyl]benzolsulfonamid und 0,4 g 1,4-Diazabicyclo[2,2,2)octan in 400 ml Xylol leitete man unter Rückfluß (Trockeneiskühlung) langsam Phosgen ein, bis eine Innentemperatur von 110°C erreicht war. Man entfernte die Kühlung und destillierte die flüchtigen Bestandteile im Wasserstrahlvakuum bei 80°C ab. Das verbleibende Sulfonylisocyanat wurde ohne weitere Reinigung umgesetzt.

### 9. N-[(n-Butylamino)carbonyl-2-(N,N-dimethylaminosulfonyl)]benzolsulfonamid

Zu einer Suspension von 44,2 g 2-[N,N-Dimethylamino)sulfonyl]benzolsulfonamid (0,17 mol) (hergestellt in analoger Weise wie 2-[N,N-Diethylamino)-sulfonyl]benzolsulfonamid in US-B-4,310,346) in 450 ml Acetonitril tropfte man bei 25°C 18,6 g n-Butylisocyanat (0,18 mol). Nach Zugabe von 25,4 g Kaliumcarbonat (0,18 mol) rührte man 3 Stunden unter Rückfluß. Nach Abkühlen auf 0°C goß man das Reaktionsgemisch auf 400 ml Eis/Wasser, stellte durch Zugabe von konz. Salzsäure einen pH-Wert von 1 ein, saugte den gebildeten Niederschlag ab, wusch diesen mit Wasser neutral und trocknete ihn im Wasserstrahlvakuum bei 40°C. Man erhielt so 60 g der Titelverbindung (99 % d. Th.).
¹H-NMR-spektrum (250 MHz, CDCl₃, int. TMS): 8,55 br (1H), 8,30 d (1H), 8,05 d (1H), 7.7-7,9 m (1H), 6,52 t (1H), 3,17 qua (2H), 2,94 s (6H), 1,43 qui (2H), 1,25 sext (2H), 0,85 t (3H).

### 10. 2-[N,N-(Dimethylamino)sulfonyl]benzolsulfonylisocyanat

Der in Beispiel 9. erhaltene Sulfonylharnstoff wurde analog zur Darstellung von 2-(Methylsulfinyl)benzolsulfonylisocyanat in das entsprechende Sulfonylisocyanat überführt.

Nachstehend sind typische Beispiele für die Synthese der Sulfonylharnstoffe I und ihrer Salze beschrieben.

### 11. 2-Nitro-1-N-[4-methoxy-6-trifluormethylpyrimidin-2-yl)aminocarbonyl]benzolsulfonamid

Eine Mischung aus 6,7 g 2-Amino-4-methoxy-6-trifluormethylpyrimidin (35 mmol) in 10 ml Dichlormethan wurde bei 25°C mit 8 g 2-Nitrobenzolsulfonylisocyanat (35 mmol) in 10 ml 1,2-Dichlorethan versetzt. Nach 14 Stunden Rühren bei 25°C wurde das Lösungsmittel im Wasserstrahlvakuum bei 40°C entfernt und der Rückstand 2 Stunden kräftig mit 50 ml Diethylether gerührt. Das Produkt wurde abgesaugt und im Wasserstrahlvakuum bei 40°C getrocknet. Man erhielt 3 g der Titelverbindung (20 % d. Th.) als farblose Kristalle mit Fp. 204 - 205°C.

### 12. Natrium[2-nitro-1-N-[(4-methoxy-6-trifluormethylpyrimidin-2-yl)aminocarbonyl]benzolsulfonamid]

Eine Suspension von 1,5 g 2-Nitro-1-N-[4-methoxy-6-trifluormethylpyrimidin-2-yl)aminocarbonyl)benzolsulfonamid (3,6 mmol) in 20 ml Methanol wurde bei 25°C mit 0,65 g (3,6 mmol) einer Lösung von Natriummethanolat (30 Gew.-%) in Methanol versetzt. Nach 10 Minuten Rühren bei 25°C wurde die homogene Lösung im Wasserstrahlvakuum bei 50°C eingeengt. Man erhielt die Titelverbindung in quantitativer Ausbeute als farblose Kristalle mit Zersetzungspunkt 164 - 166°C.

### 13. 2-[[(4-Fluor-6-methoxypyrimidin-2-yl)aminocarbonyl]aminosulfonyl]benzolsulfonsäure(N,N-dimethyl)amid

Eine Suspension von 4,0 g 2-Amino-4-fluor-6-methoxypyrimidin (28 mmol) in 30 ml Methylenchlorid wurde bei 25°C mit 8,1 g 2-(Dimethylamino)sulfonylbenzolsulfonylisocyanat (28 mmol) versetzt. Man rührte 16 Stunden bei 25°C nach und saugte das abgeschiedene Produkt ab. Zur Beseitigung von nicht umgesetztem Pyrimidin wurde der Niederschlag mit Diisopropylether kräftig gerührt. Das Produkt wurde abgesaugt, mit n-Hexan gewaschen und bei 40°C im Wasserstrahlvakuum getrocknet.

Man erhielt so 7,2 g (59 %) der Titelverbindung (Zersetzungspunkt 198°C). Aus der Mutterlauge konnte weiteres Produkt isoliert werden.
¹H-NMR-Spektrum (250 MHz, CD₃COCD₃, int. TMS, δ (ppm)): 2,91 s (6H), 4,17 s (6H), 6,24 s (1H), 7.8-8,0 m (2H), 8,04 m (1H), 8,50 m (1H), 9,63 br (1H), 12,25 br (1H).

### 14. [(4-Fluor-6-methoxypyrimidin-2-yl)aminocarbonyl]-2-methylsulfonylbenzolsulfonamid

Eine Lösung aus 2,7 g 2-Amino-4-fluor-6-methoxypyrimidin (19 mmol) in 30 ml Methylenchlorid wurde bei 25°C mit 4,7 g 2-(Methylsulfinyl)benzolsulfonylisocyanat (19 mmol) versetzt. Man rührte 16 Stunden bei 25°C nach, saugte das abgeschiedene Produkt ab, wusch mit wenig Ether und trocknete im Wasserstrahlvakuum bei 50°C. Man erhielt so 0,9 g der Titelverbindung (10 % d. Th.) mit Fp. 161 - 176°C. Aus der Mutterlauge konnte weiteres Produkt isoliert werden.

Die in der nachfolgenden Tabelle 1 genannten Wirkstoffe wurden auf analogem Herstellungsweg erhalten.

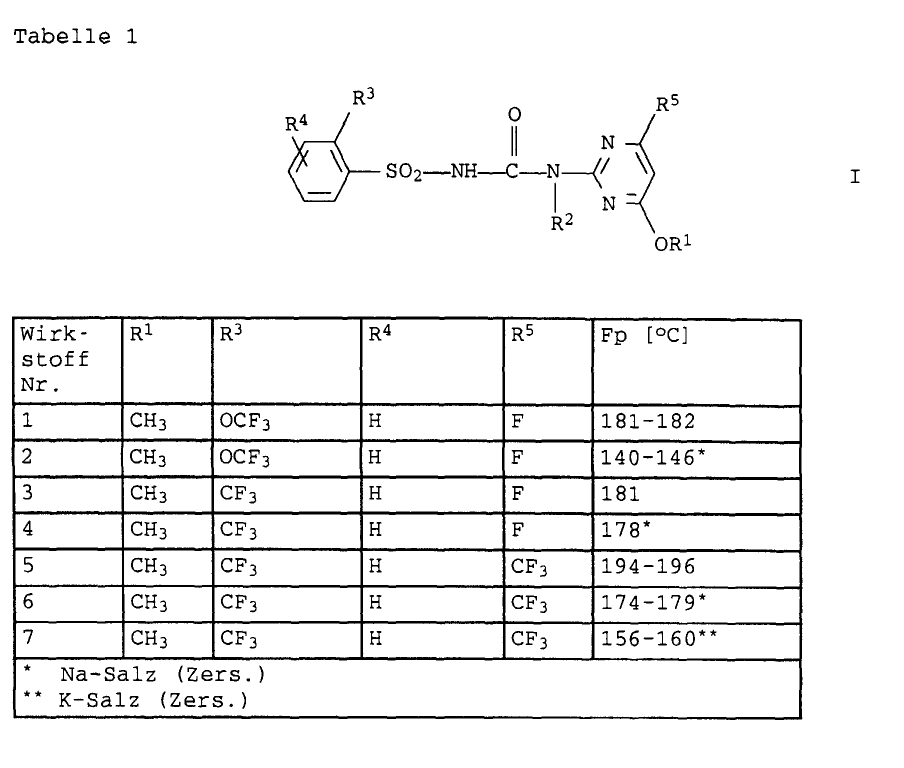

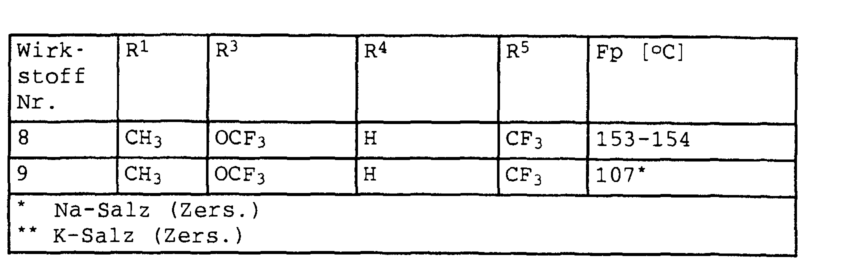

### Anwendungsbeispiele:

Die herbizide Wirkung der N-[(Pyrimidin-2-yl)aminocarbonyl]-benzolsulfonamide der Formel I auf das Wachstum der Testpflanzen wird durch folgende Gewächshausversuche gezeigt.

Als Kulturgefäße dienen Plastikblumentöpfe mit 300 cm³ Inhalt und lehmigem Sand mit etwa 3,0 % Humus als Substrat. Die Samen der Testpflanzen werden nach Arten getrennt flach eingesät.

Zum Zwecke der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,06 bzw. 0,03 kg/ha a.S. (aktive Substanz).

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| | |
| Alopecurus myosuroides | Ackerfuchsschwanz |
| Echinochloa crus-galli | Hühnerhirse |
| Setaria italica | Kolbenhirse |
| Zea mays | Mais |

Mit 0,06 bzw. 0,03 kg/ha a.S. im Nachauflaufverfahren eingesetzt lassen sich mit Beispiel Nr. 3 unerwünschte Pflanzen sehr gut bekämpfen bei gleichzeitiger Verträglichkeit in der Beispielkultur Mais.

Den Verbindungen Nr. 7 und 3 wurden die aus DE-A 27 15 786 (US 4,120,691) bekannten Vergleichssubstanzen N und O gegenübergestellt. Die in Tabellen II bis IV zusammengestellten Ergebnisse belegen die bessere herbizide Aktivität bei gleichzeitig hoher Selektivität in der Beispielkultur Winterweizen (Triticum aestivum).

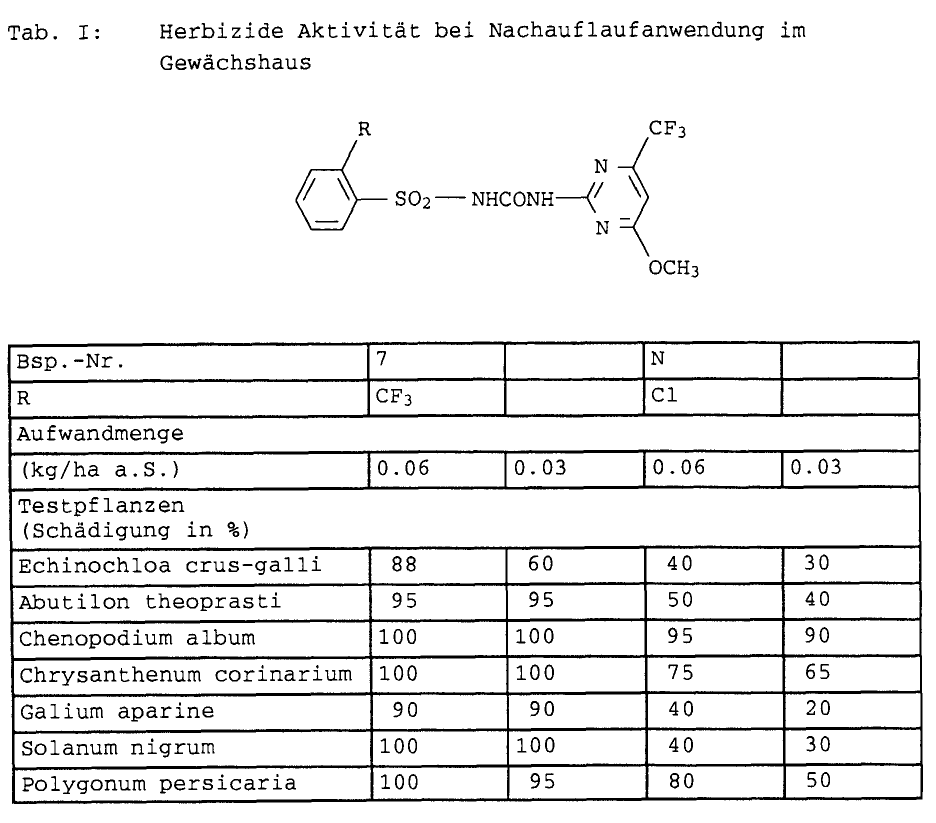

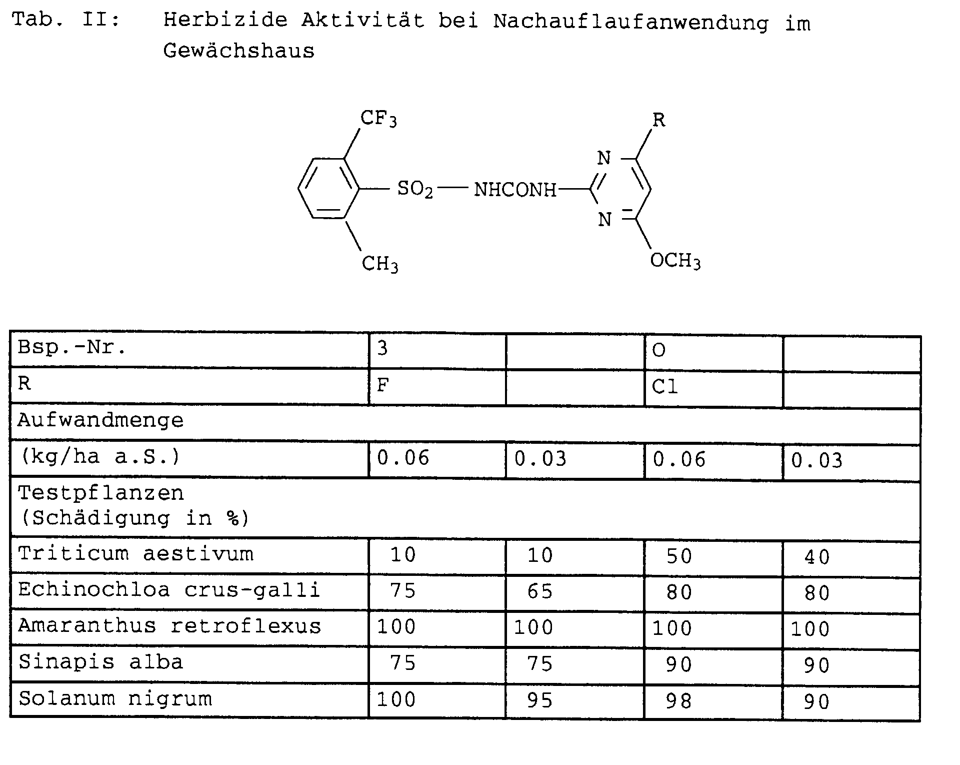

## Patentansprüche

1. N- [(Pyrimidin-2-yl)aminocarbonyl]benzolsulfonamide der allgemeinen Formel I 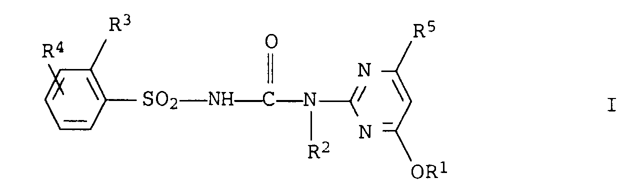 in der die Substituenten folgende Bedeutung haben:
R¹ Methyl;
R² Wasserstoff;
R³ Trifluormethyl oder Trifluormethoxy;
R⁴ Wasserstoff;
R⁵ Fluor oder Trifluormethyl
sowie deren umweltverträgliche Salze.

2. N-[(Pyrimidin-2-yl)aminocarbonyl]benzolsulfonamide der Formel I gemäß Anspruch 1, in der R¹ Methyl, R² und R⁴ Wasserstoff, R³ Trifluormethyl und R⁵ Trifluormethyl oder Fluor bedeuten.

3. N-[(Pyrimidin-2-yl)aminocarbonyl]benzolsulfonamide der Formel I gemäß Anspruch 1, in der R¹ Methyl, R² und R⁴ Wasserstoff, R³ Trifluormethoxy und R⁵ Trifluormethyl oder Fluor bedeuten.

4. Verfahren zur Herstellung der N-[(Pyrimidin-2-yl)aminocarbonyl]benzolsulfonamide der Formel I, dadurch gekennzeichnet, daß man ein Sulfonylisocyanat II 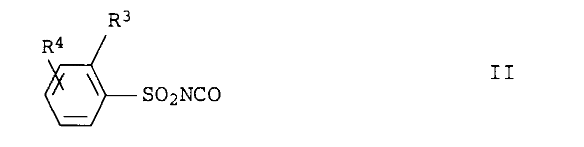 in an sich bekannter Weise in einem inerten organischen Lösungsmittel mit der ungefähr stöchiometrischen Menge eines 2-Aminopyrimidinderivats III 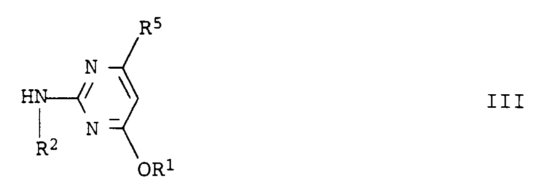 umsetzt.

5. Verfahren zur Herstellung der N-[(Pyrimidin-2-yl)amino-carbonyl]benzolsulfonamide der Formel I gemäß Anspruch 1, dadurch gekennzeichnet, daß man ein Carbamat der Formel IV 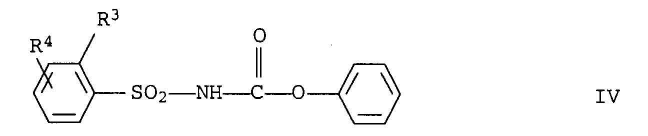 in an sich bekannter Weise in einem inerten organischen Lösungsmittel bei einer Temperatur zwischen 0 und 120°C mit ungefähr der stöchiometrischen Menge eines 2-Aminopyrimidins III umsetzt.

6. Verfahren zur Herstellung der N-[(Pyrimidin-2-yl)aminocarbonyl]benzolsulfonamide der Formel I gemäß Anspruch 1, dadurch gekennzeichnet, daß man ein entsprechendes Sulfonamid der Formel V 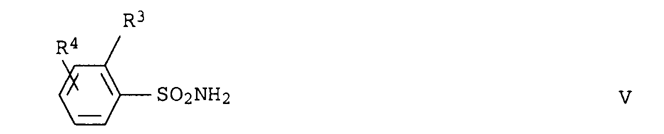 in an sich bekannter Weise in einem inerten organischen Lösungsmittel mit einem Phenylcarbamat VI 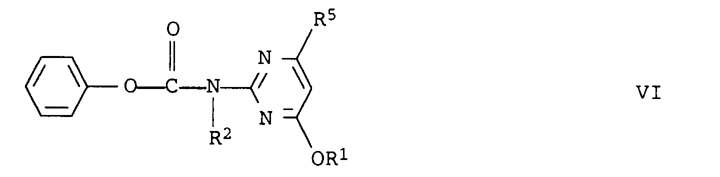 umsetzt.

7. Verfahren zur Herstellung der N-[(Pyrimidin-2-yl)aminocarbonyl]benzolsulfonamide der Formel I gemäß Anspruch 1, dadurch gekennzeichnet, daß man ein entsprechendes Sulfonamid der Formel V 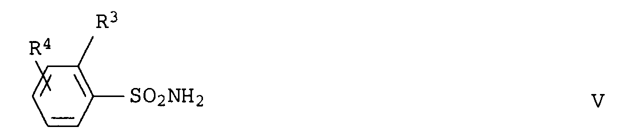 in an sich bekannter Weise in einem inerten organischen Lösungsmittel mit einem Isocyanat der Formel VII 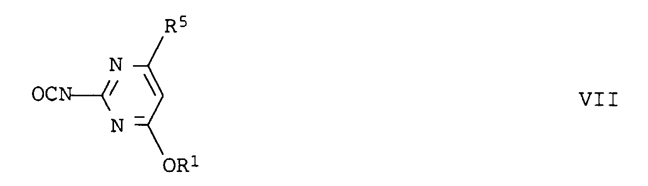 umsetzt.

8. Herbizides Mittel, enthaltend ein N-[(Pyrimidin-2-yl)aminocarbonyl]benzolsulfonamid der Formel I gemäß Anspruch 1 oder sein Salz sowie hierfür übliche Trägerstoffe.

9. Verfahren zur Bekämpfung unerwünschten Pflanzenwuchses, dadurch gekennzeichnet, daß man eine herbizid wirksame Menge eines N-[(Pyrimidin-2-yl)aminocarbonyl]benzolsulfonamids der Formel I gemäß Anspruch 1 oder eines seiner Salze auf die Pflanzen und/oder ihren Lebensraum einwirken läßt.

## Claims

1. An N-[(pyrimidin-2-yl)-aminocarbonyl]-benzenesulfonamide of the formula I 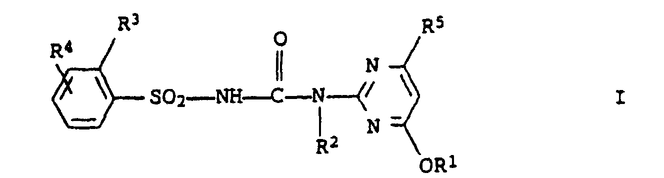 where
R¹ is methyl;
R² is hydrogen;
R³ is trifluoromethyl or trifluoromethoxy;
R⁴ is hydrogen, and
R⁵ is fluorine or trifluoromethyl,
and its environmentally compatible salts.

2. An N-[(pyrimidin-2-yl)-aminocarbonyl]-benzenesulfonamide of the formula I as claimed in claim 1, wherein R¹ is methyl, R² and R⁴ are hydrogen, R³ is trifluoromethyl and R⁵ is trifluoromethyl or fluorine.

3. An N-[(pyrimidin-2-yl)-aminocarbonyl]-benzenesulfonamide of the formula I as claimed in claim 1, wherein R¹ is methyl, R² and R⁴ are hydrogen, R³ is trifluoromethoxy and R⁵ is trifluoromethyl or fluorine.

4. A process for the preparation of an N-[(pyrimidin-2-yl)-aminocarbonyl]-benzenesulfonamide of the formula I, wherein a sulfonylisocyanate II 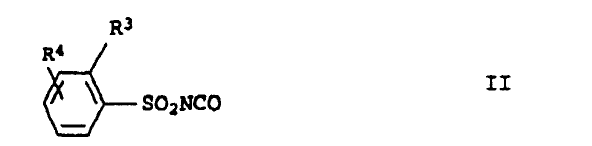 is reacted in a conventional manner, in an inert organic solvent, with about the stoichiometric amount of a 2-aminopyrimidine derivative III

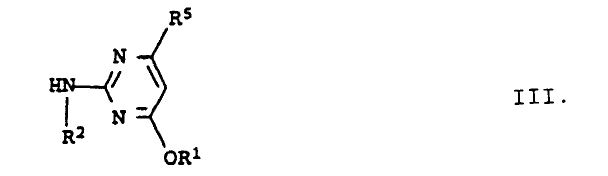

5. A process for the preparation of an N-[(pyrimidin-2-yl)-aminocarbonyl]-benzenesulfonamide of the formula I as claimed in claim 1, wherein a carbamate of the formula IV 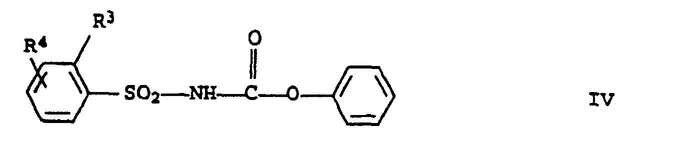 is reacted in a conventional manner, in an inert organic solvent, at from 0 to 120°C, with about the stoichiometric amount of a 2-aminopyrimidine III.

6. A process for the preparation of an N-[(pyrimidin-2-yl)-aminocarbonyl]-benzenesulfonamide of the formula I as claimed in claim 1, wherein a corresponding sulfonamide of the formula V 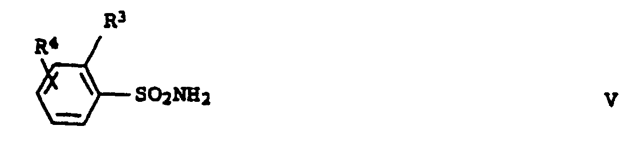 is reacted in a conventional manner, in an inert organic solvent, with a phenyl carbamate VI

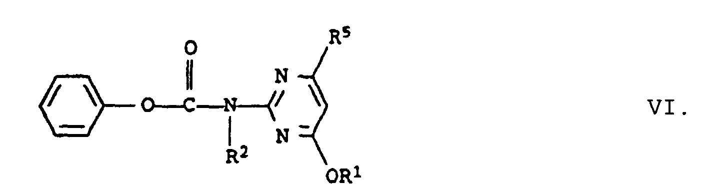

7. A process for the preparation of an N-[(pyrimidin-2-yl)-aminocarbonyl] -benzenesulfonamide of the formula I as claimed in claim 1, wherein a corresponding sulfonamide of the formula V 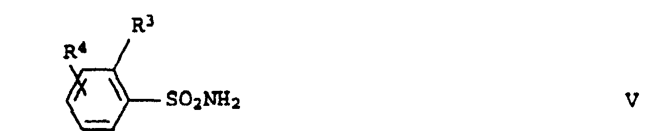 is reacted in a conventional manner, in an inert organic solvent, with an isocyanate of the formula VII

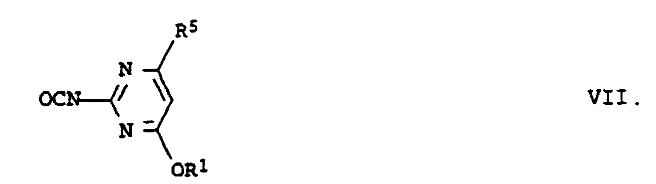

8. A herbicide containing an N-[(pyrimidin-2-yl)-aminocarbonyl]-benzenesulfonamide of the formula I as claimed in claim 1 or its salt and conventional carriers.

9. A method for controlling undesirable plant growth, wherein a herbicidal amount of an N-[(pyrimidin-2-yl)-aminocarbonyl]-benzenesulfonamide of the formula I as claimed in claim 1 or of one of its salts is allowed to act on the plants and/or their habitat.

## Revendications

1. N-[(pyrimidine-2 -yl)aminocarbonyl]benzènesulfonamides de formule générale I 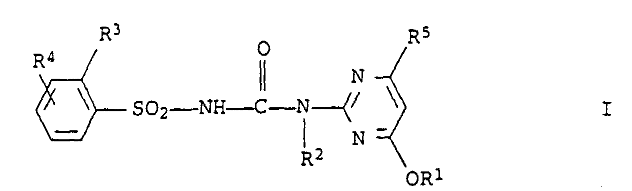 dans laquelle les substituants ont les significations suivantes:
R¹ méthyle,
R² hydrogène,
R³ trifluorométhyle ou trifluorométhoxy,
R⁴ hydrogène,
R⁵ fluoro ou trifluorométhyle,
ainsi que leurs sels compatibles avec l'environnement.

2. N-[(pyrimidine-2-yl)aminocarbonyl]benzènesulfonamides de formule I selon la revendication 1, dans laquelle R¹ est un groupe méthyle, R² et R⁴ sont l'hydrogène, R³ est un groupe trifluorométhyle et R⁵ représente un groupe trifluorométhyle ou fluoro.

3. N-[(pyrimidine-2-yl)aminocarbonyl]benzènesulfonamides de formule I selon la revendication 1, dans laquelle R¹ est un groupe méthyle, R² et R⁴ sont l'hydrogène, R³ est un groupe trifluorométhoxy et R⁵ représente un groupe trifluorométhyle ou fluoro.

4. Procédé pour la préparation des N-[(pyrimidine-2-yl)aminocarbonyl]benzènesulfonamides de formule I, caractérisé par le fait qu'on fait réagir un sulfonylisocyanate II 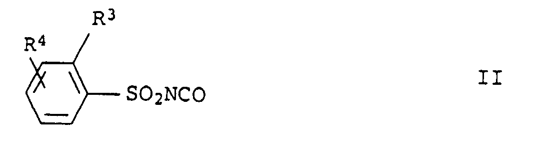 d'une manière connue en soi, dans un solvant organique inerte, avec la quantité approximativement stoechiométrique d'un dérivé de 2-aminopyrimidine III

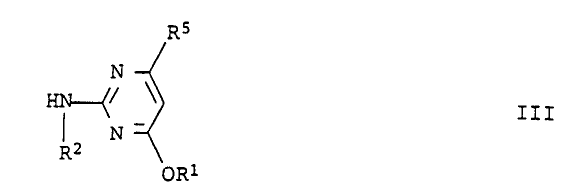

5. Procédé pour la préparation des N-[(pyrimidine-2-yl)aminocarbonyl]benzènesulfonamides de formule I selon la revendication 1, caractérisé par le fait qu'on fait réagir un carbamate de formule IV 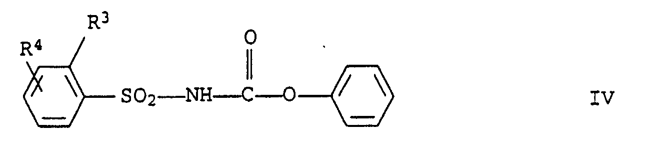 d'une manière connue en soi, dans un solvant organique inerte, à une température comprise entre 0 et 120°C, avec la quantité approximativement steochiométrique d'une 2-aminopyrimidine III.

6. Procédé pour la préparation des N-[(pyrimidine-2-yl)aminocarbonyl]benzènesulfonamides de formule I selon la revendication 1, caractérisé par le fait qu'on fait réagir un sulfonamide correspondant de formule V 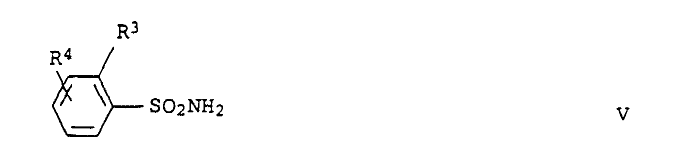 d'une manière connue en soi, dans un solvant organique inerte, avec un phénylcarbamate VI

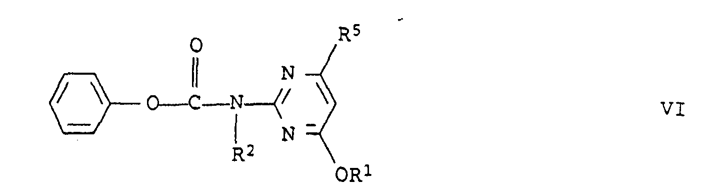

7. Procédé pour la préparation des N-[(pyrimidine-2-yl)aminocarbonyl]benzènesulfonamides de formule I selon la revendication 1, caractérisé par le fait qu'on fait réagir un sulfonamide correspondant de formule V 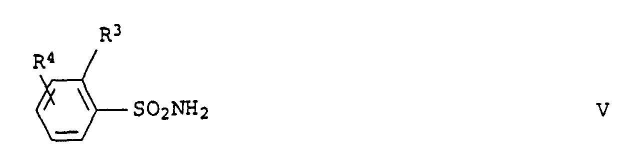 d'une manière connue en soi, dans un solvant organique inerte, avec un isocyanate de formule VII

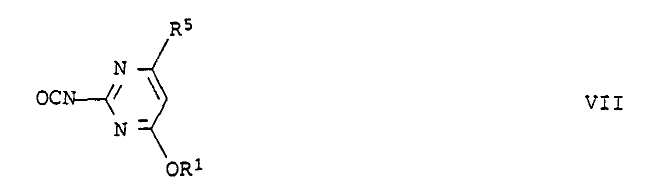

8. Agent herbicide, contenant un N-[(pyrimidine-2-yl)aminocarbonyl]benzènesulfonamide de formule I selon la revendication 1 ou son sel, ainsi que les supports classiques pour cet usage.

9. Procédé pour lutter contre la croissance des plantes parasites, caractérisé par le fait qu'on fait agir sur les plantes et/ou leur espace vital une quantité efficace du point de vue herbicide d'un N-[(pyrimidine-2-yl)aminocarbonyl]benzènesulfonamide de formule I selon la revendication 1 ou un de ses sels.
